(19) Europäisches Patentamt
European Patent Office
Office européen des brevets

(11) **EP 4 736 895 A1**

(12) **EUROPEAN PATENT APPLICATION**
published in accordance with Art. 153(4) EPC

(43) Date of publication:
**06.05.2026 Bulletin 2026/19**

(21) Application number: **24832266.1**

(22) Date of filing: **23.05.2024**

(51) International Patent Classification (IPC):
**A61L 27/12** (2006.01)    **A61L 27/04** (2006.01)

(52) Cooperative Patent Classification (CPC):
**A61L 27/04; A61L 27/12**

(86) International application number:
**PCT/KR2024/007005**

(87) International publication number:
**WO 2025/005483 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.06.2023 KR 20230084738**

(71) Applicant: **Osstemimplant Co., Ltd.
Gangseo-gu
Seoul 07789 (KR)**

(72) Inventors:
• **JO, Hee Ryeong
Seoul 07789 (KR)**
• **PARK, Hyung Joon
Seoul 07789 (KR)**

(74) Representative: **Germain Maureau
12, rue Boileau
69006 Lyon (FR)**

(54) **LOW-CRYSTALLINE CALCIUM PHOSPHATE-BASED ANTIBACTERIAL BONE GRAFT MATERIAL AND METHOD FOR PREPARING SAME**

(57)    One embodiment of the present disclosure provides a method for preparing a low-crystalline calcium phosphate-based antibacterial bone graft material, the method comprising: (a) synthesizing calcium phosphate by adding a calcium precursor and a phosphate precursor all at once; (b) introducing the calcium phosphate into an antibacterial material precursor solution to perform a reaction; (c) freeze-drying a product of step (b); (d) mixing a product of step (c) with a pore-forming agent and press-molding the mixture to prepare a molded body; and (e) removing the pore-forming agent of the molded body in a solvent at a temperature equal to or higher than the boiling point of the solvent, followed by drying.

FIG. 1

**Description**

[Technical Field]

The present disclosure relates to a low-crystalline calcium phosphate-based antibacterial bone graft material and a method for preparing the same.

[Background Art]

A bone graft material is a material for regenerating bone absorbed or destroyed due to factors such as disease, trauma, and aging.

For example, in dental treatments such as implant placement surgery or periodontal surgery, osteoplasty is sometimes performed to artificially create alveolar bone using a bone graft material.

Bone graft materials play a role in stimulating bone growth, and their mechanisms of action can be broadly classified into osteoconduction, osteoinduction, and osteogenesis. Osteoconduction is a mechanism in which osteoblasts migrate from surrounding bone tissue to form bone, and bone tissue or differentiated mesenchymal cells are essential at the graft site.

Osteoinduction refers to a mechanism that influences the differentiation of undifferentiated mesenchymal cells into osteoprogenitor cells to form bone tissue. Osteogenesis refers to a mechanism of generating bone tissue by cells inside the graft material.

Bone graft materials can be classified into autogenous bone, allogenic bone, xenograft, and synthetic bone (alloplastic bone) graft materials according to their origin.

Autogenous bone graft materials use bone tissue harvested from a recipient subject, possess all of osteogenic, osteoconductive, and osteoinductive capabilities, and are the only ones among existing bone graft materials that have osteogenic capability.

However, there are limitations in that additional surgery is required for harvesting bone tissue, which entails costs and time, and above all, the amount of available bone is limited.

Allogenic bone graft materials harvest bone tissue from a cadaver and are generally obtained by receiving donated bone from a deceased person.

Since they are graft materials obtained from the genetically same species, they have equivalent healing capabilities and are excellent in bone replacement effects due to absorbability and osteogenic capability.

Xenograft graft materials are harvested from individuals of different species, and hydroxyapatite obtained mainly from bovine or porcine sources is used.

Various components including calcium phosphate-based ceramics are used as synthetic bone graft materials, but they are generally known to be inferior in osteogenic capability and bone quality compared to autogenous or allogenic bone graft materials.

Calcium phosphate-based biomaterials mainly used as synthetic bone graft materials include hydroxyapatite (HA) and tricalcium phosphate (TCP).

However, hydroxyapatite has a limitation in that it is non-absorbable and remains in the body for a long period of time, and tricalcium phosphate has a drawback in that the rate of bone formation and the degradation rate of the graft material become unbalanced.

In addition, if the site of bone graft procedure becomes infected, bone recovery is difficult and bone defects may accompany, requiring complex recovery means. In particular, in the case of dental bone graft materials, it is known that the possibility of bacterial infection is high due to the intraoral environment.

Accordingly, there is a need for the development of a bone graft material that is a synthetic bone graft material having absorbability and osteogenic capability at a level similar to that of allogenic bone, and can actively control infection by inhibiting the growth of bacteria by the graft material itself.

[Disclosure]

[Technical Problem]

The present disclosure is intended to solve the problems of the related art described above, and an object of the present disclosure is to provide a low-crystalline calcium phosphate-based antibacterial bone graft material having excellent antibacterial properties and osteogenic capability, and a method for preparing the same.

[Technical Solution]

According to an aspect, there is provided a method for preparing a low-crystalline calcium phosphate-based antibacterial bone graft material, the method comprising: (a) synthesizing calcium phosphate by adding a calcium precursor and a phosphate precursor all at once; (b) introducing the calcium phosphate into an antibacterial material precursor solution to perform a reaction; (c) freeze-drying a product of step (b); (d) mixing a product of step (c) with a pore-forming agent and press-molding the mixture to prepare a molded body; and (e) removing the pore-forming agent of the molded body in a solvent at a temperature equal to or higher than the boiling point of the solvent, followed by drying.

In an embodiment, the calcium precursor may include at least one selected from the group consisting of calcium nitrate, calcium chloride, calcium fluoride, calcium iodide, calcium acetate, ammonium carbonate, and ammonium bicarbonate.

In an embodiment, the phosphate precursor may include at least one selected from the group consisting of phosphorus oxide, ammonium phosphate monobasic, ammonium phosphate dibasic, ammonium phosphate tribasic, sodium phosphate monobasic, sodium phosphate dibasic, sodium phosphate tribasic, and potassium phosphate.

In an embodiment, in step (a), a molar ratio of the calcium precursor and the phosphate precursor may be 1: 1.5 to 3.

In an embodiment, step (a) may be a wet precipitation method in which each precursor is dissolved in distilled water and mixed.

In an embodiment, the antibacterial material may include at least one selected from the group consisting of silver (Ag), magnesium (Mg), gallium (Ga), zinc (Zn), and copper (Cu).

In an embodiment, the reaction of step (b) may be performed at a temperature of 15 to 25°C for 10 to 20 hours.

In an embodiment, in step (d), a weight ratio of the product of step (c) and the pore-forming agent may be 1:1 to 1.5.

In an embodiment, an average particle diameter of the pore-forming agent may be 100 to 500 $\mu$m.

In an embodiment, after step (e), the method may further comprise (f) heat-treating the molded body at a temperature of 250°C or lower.

In an embodiment, a content of the antibacterial material in the low-crystalline calcium phosphate-based antibacterial bone graft material may be 0.2 to 1.8% by weight.

In an embodiment, the content of the antibacterial material in the low-crystalline calcium phosphate-based antibacterial bone graft material may be 0.5 to 0.9% by weight.

According to another aspect, there is provided a low-crystalline calcium phosphate-based antibacterial bone graft material comprising an antibacterial material, wherein the bone graft material includes at least one of grooves or pores having an average diameter of 100 to 500 $\mu$m, has a BET specific surface area of 100 $m^2/g$ or more, includes both crystalline apatite and amorphous calcium phosphate, and has a crystallinity of 25% or less.

In an embodiment, the antibacterial material may include at least one selected from the group consisting of silver (Ag), magnesium (Mg), gallium (Ga), zinc (Zn), and copper (Cu).

In an embodiment, a content of the antibacterial material may be 0.2 to 1.8% by weight relative to total elements constituting the bone graft material.

In an embodiment, the content of the antibacterial material may be 0.5 to 0.9% by weight relative to the total elements constituting the bone graft material.

[Advantageous Effects]

The method for preparing a low-crystalline calcium phosphate-based antibacterial bone graft material according to an aspect of the present disclosure can be applied to the production of a low-crystalline calcium phosphate-based antibacterial bone graft material having excellent antibacterial properties and osteogenic capability.

In addition, the low-crystalline calcium phosphate-based antibacterial bone graft material according to another aspect of the present disclosure has excellent antibacterial properties, thereby lowering the risk of infection due to bacterial proliferation, and at the same time, has absorbability and osteoconductivity, and can exhibit osteogenic capability equivalent to that of allogenic bone.

The effects of an aspect of the present disclosure are not limited to the above-described effects, and should be understood to include all effects inferable from the configurations described in the detailed description or claims of the present disclosure.

[Description of Drawings]

FIG. 1 shows results of antibacterial property evaluation of bone graft materials according to Examples and Comparative Examples of the present disclosure.

FIG. 2 shows results of cytotoxicity evaluation of bone graft materials according to Examples and Comparative Examples of the present disclosure.

FIGS. 3 and 4 show results of XRD analysis of bone graft materials according to Examples and Comparative Examples of the present disclosure.

FIG. 5 is a dispersion graph of EDS measurement values of a bone graft material according to an Example of the present disclosure.

FIG. 6 is an SEM image of a bone graft material according to an Example of the present disclosure.

FIG. 7 shows results of BET specific surface area analysis of bone graft materials according to Examples and Comparative Examples of the present disclosure.

[Detailed Discription]

Hereinafter, embodiments of the present disclosure will be described with reference to the accompanying drawings.

However, the matters described in the present disclosure may be implemented in various different forms, and thus are not limited to the examples described herein.

In order to clearly describe an aspect of the present disclosure in the drawings, parts irrelevant to the description are omitted, and similar reference numerals are assigned to similar parts throughout the specification.

Throughout the specification, when a part is referred to as being "connected" to another part, this includes not only the case where it is "directly connected" but also the case where it is "indirectly connected" with another member interposed therebetween. Also, when a part is referred to as "including" a component, this means that it may further include other components rather than excluding other components, unless specifically stated to the contrary.

When a range of numerical values is described in the present disclosure, the value has the precision of the significant figures provided according to the standard rules in chemistry for significant figures, unless the specific range thereof is stated otherwise. For example, 10 includes a range of 5.0 to 14.9, and the number 10.0 includes a range of 9.50 to 10.49.

Hereinafter, an embodiment of the present disclosure will be described in detail with reference to the accompanying drawings.

Method for preparing low-crystalline calcium phosphate-based antibacterial bone graft material

A method for preparing a low-crystalline calcium phosphate-based antibacterial bone graft material according to an aspect of the present disclosure comprises: (a) synthesizing calcium phosphate by adding a calcium precursor and a phosphate precursor all at once; (b) introducing the calcium phosphate into an antibacterial material precursor solution to perform a reaction; (c) freeze-drying a product of step (b); (d) mixing a product of step (c) with a pore-forming agent and press-molding the mixture to prepare a molded body; and (e) removing the pore-forming agent of the molded body in a solvent at a temperature equal to or higher than the boiling point of the solvent, followed by drying.

As used herein, the term "low-crystalline (or low crystallinity)" means that the proportion of the apatite structure, which is a crystalline phase, among the phases constituting the bone graft material is lower than usual.

For example, the low crystallinity may mean that the crystallinity is half or less, or 25% or less, but is not limited thereto, and may mean a low-crystalline component recognized within a typical technical scope in the art.

The "crystallinity" can be obtained through X-ray diffraction (XRD) analysis according to ISO 13779-3 standard.

Step (a) is a step of preparing a raw material for the low-crystalline calcium phosphate-based bone graft material.

The calcium precursor may include at least one selected from the group consisting of calcium nitrate, calcium chloride, calcium fluoride, calcium iodide, calcium acetate, ammonium carbonate, and ammonium bicarbonate, but is not limited thereto.

The phosphate precursor may include at least one selected from the group consisting of phosphorus oxide, ammonium phosphate monobasic, ammonium phosphate dibasic, ammonium phosphate tribasic, sodium phosphate monobasic, sodium phosphate dibasic, sodium phosphate tribasic, and potassium phosphate, but is not limited thereto.

In step (a), a molar ratio of the calcium precursor and the phosphate precursor may be 1:1.5 to 3.

For example, it may be 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9, 1:3, or a range between any two of these values.

If it is outside the above range, the reaction may proceed inefficiently, or it may be difficult to prepare a bone graft material having a desired composition.

Step (a) may be a wet precipitation method in which each precursor is dissolved in distilled water and mixed, but is not limited thereto.

The wet precipitation method may be performed in a dropwise manner to maintain vacuum conditions, but since the preparation method does not require vacuum conditions as an essential condition, calcium phosphate can be synthesized simply by adding all at once.

The calcium phosphate may be aged for 1 to 24 hours before step (b), and then filtered and washed for use.

Step (b) is a step of imparting antibacterial properties to the low-crystalline calcium phosphate-based bone graft material.

The antibacterial material precursor solution may include a precursor of an antibacterial material and distilled water, but is not limited thereto.

The antibacterial material may include at least one selected from the group consisting of silver (Ag), magnesium (Mg), gallium (Ga), zinc (Zn), and copper (Cu), but is not limited thereto.

The reaction of step (b) may be an ion exchange reaction. For example, the antibacterial material may be present by being substituted in the crystal of the calcium phosphate. The reaction of step (b) may be performed at a temperature of 15 to 25°C for 10 to 20 hours.

For example, it may be performed at a temperature of 15°C, 16°C, 17°C, 18°C, 19°C, 20°C, 21°C, 22°C, 23°C, 24°C, 25°C, or a temperature between any two of these temperatures, for 10 hours, 11 hours, 12 hours, 13 hours, 14 hours, 15 hours, 16 hours, 17 hours, 18 hours, 19 hours, 20 hours, or a time between any two of these times.

The product of step (b) may be washed with ammonia and then filtered for use before step (c).

Step (c) is a step of preparing the product of step (b) into a powder.

The freeze-drying may be a method of sublimating and removing moisture by reducing atmospheric pressure at a low temperature of room temperature or lower.

Such freeze-drying conditions are not limited as long as they are temperatures and pressures at which ice can sublime.

The freeze-drying may be performed for 36 to 48 hours. Using freeze-drying can minimize damage or denaturation of the bone graft material.

If high-temperature drying is performed in step (c), the proportion of the apatite structure, which is a crystalline phase, increases, making it difficult to realize desired properties. Step (d) is a step of molding the bone graft material.

The pore-forming agent may be sodium chloride (NaCl), but is not limited thereto. The pore-forming agent may function as a template forming a skeleton of the bone graft material during molding and hydrothermal treatment.

In step (d), a weight ratio of the product of step (c) and the pore-forming agent may be 1:1 to 1.5.

For example, it may be 1:1, 1:1.05, 1:1.1, 1:1.15, 1:1.2, 1:1.25, 1:1.3, 1:1.35, 1:1.4, 1:1.45, 1:1.5, or a weight ratio between any two of these weight ratios.

If the weight ratio of the product of step (c) and the pore-forming agent is outside the above range, it may be difficult to remove the pore-forming agent, or pores or grooves of the bone graft material may not be properly formed, resulting in a decrease in bone regeneration and osteoconductivity.

An average particle diameter of the pore-forming agent may be 100 to 500 μm. For example, it may be 100 μm, 120 μm, 140 μm, 160 μm, 180 μm, 200 μm, 220 μm, 240 μm, 260 μm, 280 μm, 300 μm, 320 μm, 340 μm, 360 μm, 380 μm, 400 μm, 420 μm, 440 μm, 460 μm, 480 μm, 500 μm, or a range between any two of these values. Regions from which the pore-forming agent is removed may form grooves or pores of the bone graft material.

Therefore, if the average particle diameter of the pore-forming agent satisfies the above range, a low-crystalline calcium phosphate-based antibacterial bone graft material including at least one of grooves or pores having an average diameter of 100 to 500 μm can be prepared.

Step (e) is a step of removing the pore-forming agent acting as a template and imparting desired properties to the bone graft material.

Step (e) may include, for example, a hydrothermal treatment process of removing the pore-forming agent present inside and outside the molded body in water at 100°C, which is a temperature equal to or higher than the boiling point of the solvent.

Thereafter, moisture may be removed by drying the hydrothermally treated molded body under conditions of 250°C or lower, 225°C or lower, 200°C or lower, 175°C or lower, 150°C or lower, 125°C or lower, or 100°C or lower.

After step (e), the method may further comprise (f) heat-treating the molded body at a temperature of 250°C or lower.

The heat treatment may be performed at a temperature of, for example, 250°C, 225°C, 200°C, 175°C, 150°C, 125°C, 100°C, 75°C, 50°C, or a range between any two of these values.

If the heat treatment temperature exceeds the above range, the specific surface area may decrease and crystallinity may increase due to densification caused by grain growth. As a result, the absorbability and osteogenic capability of the bone graft material may decrease.

That is, by controlling the temperature of the heat treatment process, the specific surface area of the bone graft material increases, facilitating the binding and storage of proteins and growth factors, and blood wettability and blood clot formation can be enhanced to increase the adhesion of cells related to osteogenesis.

Finally, osseointegration between the graft material and bone tissue can be promoted. In addition, the absorption rate of the bone graft material can be controlled by controlling the temperature of the heat treatment process.

Performing heat treatment at a relatively high temperature may increase crystallinity, thereby slowing the absorption rate.

A content of the antibacterial material in the low-crystalline calcium phosphate-based antibacterial bone graft material may be 0.2 to 1.8% by weight.

For example, it may be 0.2 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.6 wt%, 1.7 wt%, 1.8 wt%, or a value between any two of these values.

If the content of the antibacterial material is less than the above range, the antibacterial property of the bone graft material may decrease, increasing the risk of infection due to bacterial proliferation.

If the content of the antibacterial material exceeds the above range, a secondary phase may be generated in addition to the low-crystalline calcium phosphate, which may degrade physical properties.

In one example, the content of the antibacterial material in the low-crystalline calcium phosphate-based antibacterial bone graft material may be 0.5 to 0.9% by weight, but is not limited thereto.

If the content of the antibacterial material is less than the above range, the antibacterial property of the bone graft material may decrease.

If the content of the antibacterial material exceeds the above range, the antibacterial material may be crystallized and present in the form of particles, and a washing process for removing antibacterial material particles that are not substituted into the lattice may be additionally performed.

Low-crystalline calcium phosphate-based antibacterial bone graft material

A low-crystalline calcium phosphate-based antibacterial bone graft material according to another aspect of the present disclosure may include an antibacterial material, include at least one of grooves or pores having an average diameter of 100 to 500 $\mu$m, have a BET specific surface area of 100 $m^2$/g or more, include both crystalline apatite and amorphous calcium phosphate, and have a crystallinity of 25% or less.

The antibacterial material may include at least one selected from the group consisting of silver (Ag), magnesium (Mg), gallium (Ga), zinc (Zn), and copper (Cu), but is not limited thereto.

A content of the antibacterial material may be 0.2 to 1.8% by weight relative to total elements constituting the bone graft material.

For example, it may be 0.2 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.6 wt%, 1.7 wt%, 1.8 wt%, or a value between any two of these values.

If the content of the antibacterial material is less than the above range, the antibacterial property of the bone graft material decreases, which may increase the risk of infection due to bacterial proliferation, and if it exceeds the above range, a secondary phase may be generated in addition to the low-crystalline calcium phosphate, which may degrade physical properties.

In one example, the content of the antibacterial material may be 0.5 to 0.9% by weight relative to the total elements constituting the bone graft material, but is not limited thereto.

If the content of the antibacterial material is less than the above range, the antibacterial property of the bone graft material may decrease, and if it exceeds the above range, the antibacterial material may be crystallized and present in the form of particles.

The low-crystalline calcium phosphate-based antibacterial bone graft material may have antibacterial activity against single oral bacteria such as Streptococcus oralis, Porphyromonas gingivalis, and Fusobacterium nucleatum, or complex bacteria including a combination thereof.

The low-crystalline calcium phosphate-based antibacterial bone graft material may exhibit a bacterial growth inhibition effect of 90% or more compared to a low-crystalline calcium phosphate-based bone graft material not containing an antibacterial material. The low-crystalline calcium phosphate-based antibacterial bone graft material may include at least one of grooves or pores concave toward the center of the particle.

Such grooves or pores allow blood cells, osteoblasts, osteoclasts, proteins, or growth factors to be loaded on the bone graft material particles, thereby promoting angiogenesis and new bone growth.

The grooves or pores of the low-crystalline calcium phosphate-based antibacterial bone graft material may have an average diameter of 100 to 500 $\mu$m, and the average diameter may be, for example, 100 $\mu$m, 120 $\mu$m, 140 $\mu$m, 160 $\mu$m, 180 $\mu$m, 200 $\mu$m, 220 $\mu$m, 240 $\mu$m, 260 $\mu$m, 280 $\mu$m, 300 $\mu$m, 320 $\mu$m, 340 $\mu$m, 360 $\mu$m, 380 $\mu$m, 400 $\mu$m, 420 $\mu$m, 440 $\mu$m, 460 $\mu$m, 480 $\mu$m, 500 $\mu$m, or a range between any two of these values.

If the average diameter of the grooves or pores is outside the above range, the osteogenic capability of the bone graft material may decrease, or the specific surface area or crystallinity may deviate from a desired range.

As used herein, the term "specific surface area" refers to a specific surface area determined by the BET (Brunauer, Emmett and Teller) method, and is a value estimated by measuring the amount of gas (e.g., nitrogen) physically adsorbed on the surface of a sample and calculating the area required for the gas to be adsorbed on a smooth surface. The low-crystalline calcium phosphate-based bone graft material may have a BET specific surface area of 100 $m^2/g$ or more.

For example, it may be 100 $m^2/g$, 102 $m^2/g$, 104 $m^2/g$, 106 $m^2/g$, 108 $m^2/g$, 110 $m^2/g$, 112 $m^2/g$, 114 $m^2/g$, 116 $m^2/g$, 118 $m^2/g$, 120 $m^2/g$, 122 $m^2/g$, 124 $m^2/g$, 126 $m^2/g$, 128 $m^2/g$, 130 $m^2/g$, 132 $m^2/g$, 134 $m^2/g$, 136 $m^2/g$, 138 $m^2/g$, 140 $m^2/g$, 142 $m^2/g$, 144 $m^2/g$, 146 $m^2/g$, 148 $m^2/g$, 150 $m^2/g$, or a range between any two of these values, but is not limited thereto.

If the BET specific surface area is less than 100 $m2/g$, it may be difficult for blood cells, osteoblasts, osteoclasts, proteins, or various growth factors to bind and be stored.

On the other hand, if the BET specific surface area satisfies the above range, blood clot formation is facilitated, and blood wettability is excellent, so that adhesion of osteogenesis-related cells is excellent, thereby promoting osseointegration between the graft material and bone tissue.

Conventional synthetic bone has a disadvantage of a small surface area because particles grow and become densified in a high-temperature sintering process.

On the other hand, it is known that natural bone has a large surface area and excellent osseointegration due to characteristics of its microstructure.

The bone graft material according to an aspect of the present disclosure is a synthetic bone but has a high surface area, and thus can have excellent osseointegration.

The "crystalline apatite" may refer to a hexagonal calcium phosphate mineral. For example, the calcium phosphate mineral may be at least one selected from the group consisting of hydroxyapatite (HA), fluorapatite, chlorapatite, and carbonate apatite, but is not limited thereto.

For example, the crystalline apatite may further include inorganic ions.

The inorganic ions may be at least one selected from the group consisting of $Mg^{2+}$, $Sr^{2+}$, $Ba^{2+}$, $Mn^{2+}$, $Zn^{2+}$, $Na^+$, $K^+$, $Ag^+$, $Ga^{3+}$, $VO_4^{3-}$, $SO_4^{2-}$, $CO_3^{2-}$, $SiO_4^{4-}$, $F^-$, and $Cl^-$, but are not limited thereto.

The inorganic ions may be added during a process of synthesizing calcium phosphate from a calcium precursor and a phosphate precursor, but are not limited thereto.

The bone graft material may have a crystallinity of 25% or less. For example, it may be 25%, 24.5%, 24%, 23.5%, 23%, 22.5%, 22%, 21.5%, 21%, 20.5%, 20%, 19.5%, 19%, 18.5%, 18%, 17.5%, 17%, 16.5%, 16%, 15.5%, 15%, 14.5%, 14%, 13.5%, 13%, 12.5%, or a range between any two of these values, but is not limited thereto.

If the crystallinity of the bone graft material deviates from the above range, the absorption rate may become excessively fast, or it may not have absorbability.

Hereinafter, Examples of the present disclosure will be described in more detail. However, the following experimental results describe only representative experimental results among the Examples, and the scope and content of the present disclosure should not be interpreted as being reduced or limited by the Examples, etc.

Each effect of various embodiments of the present disclosure not explicitly presented below will be specifically described in the corresponding section.

Example 1

A calcium ion solution was prepared by dissolving 0.3 M of calcium nitrate tetrahydrate ($Ca(NO_3)_2 \cdot 4H_2O$), which is a calcium precursor, in 250 ml of distilled water ($H_2O$).

A phosphate ion solution was prepared by dissolving 0.6 M of ammonium phosphate dibasic (($NH_4)_2HPO_4$), which is a phosphate precursor, in 500 ml of distilled water adjusted to a pH of 9.5 to 11.5 with ammonia water.

A hydroxyapatite precipitate was prepared by mixing the calcium ion solution and the phosphate ion solution.

After aging at 22°C for 6 hours, the hydroxyapatite precipitate was filtered and washed using a suction filter, a vacuum pump, and filter paper.

A silver precursor solution was prepared by dissolving silver nitrate ($AgNO_3$), which is a silver (Ag) precursor, in 250 ml of distilled water ($H_2O$) so that a silver ion content of a final product, a bone graft material, was 0.2% by weight.

The filtered hydroxyapatite precipitate was introduced into the silver precursor solution, and then an ion exchange reaction was performed at 19°C for 16 hours.

The product of the ion exchange reaction was washed with ammonia and then filtered using a suction filter, a vacuum pump, and filter paper.

The filtered material was frozen and then freeze-dried for 36 to 48 hours to prepare a silver-hydroxyapatite powder.

Sodium chloride (NaCl) as a pore-forming agent was sieved to a size of 100 to 500 $\mu$m, and the silver-hydroxyapatite powder and the sieved sodium chloride were dry-mixed in a weight ratio of 1:1 to 1.5.

The mixed powder was placed in a mold and pressurized to prepare a molded body. The prepared molded body was placed in distilled water and heated for 2 to 4 hours to remove sodium chloride in the molded body.

The hydrothermally treated molded body was dried at 100°C for 12 to 24 hours, and the dried molded body was pulverized to 0.25 to 2 mm to prepare particles.

Impurities were removed by placing the particles in a sieve, immersing them in distilled water, and flowing distilled water for 4 to 8 hours.

The washed particles were dried at 110°C for 12 to 24 hours to prepare a bone graft material.

Example 2

A bone graft material was prepared in the same manner as in Example 1, except that the silver precursor solution was prepared by dissolving silver nitrate so that the silver ion content of the final product, the bone graft material, was 0.3% by weight.

Example 3

A bone graft material was prepared in the same manner as in Example 1, except that the silver precursor solution was prepared by dissolving silver nitrate so that the silver ion content of the final product, the bone graft material, was 0.4% by weight.

Example 4

A bone graft material was prepared in the same manner as in Example 1, except that the silver precursor solution was prepared by dissolving silver nitrate so that the silver ion content of the final product, the bone graft material, was 0.6% by weight.

Example 5

A bone graft material was prepared in the same manner as in Example 1, except that the silver precursor solution was prepared by dissolving silver nitrate so that the silver ion content of the final product, the bone graft material, was 0.7% by weight.

Example 6

A bone graft material was prepared in the same manner as in Example 1, except that the silver precursor solution was prepared by dissolving silver nitrate so that the silver ion content of the final product, the bone graft material, was 1.0% by weight.

Example 7

A bone graft material was prepared in the same manner as in Example 1, except that the silver precursor solution was prepared by dissolving silver nitrate so that the silver ion content of the final product, the bone graft material, was 1.5% by weight.

Comparative Example 1

A calcium ion solution was prepared by dissolving 0.3 M of calcium nitrate tetrahydrate ($Ca(NO_3)_2 \cdot 4H_2O$), which is a calcium precursor, in 250 ml of distilled water ($H_2O$).

A phosphate ion solution was prepared by dissolving 0.6 M of ammonium phosphate dibasic (($NH_4)_2HPO_4$), which is a phosphate precursor, in 500 ml of distilled water adjusted to a pH of 9.5 to 11.5 with ammonia water.

A hydroxyapatite precipitate was prepared by mixing the calcium ion solution and the phosphate ion solution.

After aging at 22°C for 6 hours, the hydroxyapatite precipitate was filtered and washed using a suction filter, a vacuum pump, and filter paper.

The filtered hydroxyapatite precipitate was frozen and then freeze-dried for 36 to 48 hours to prepare a hydroxyapatite powder.

Sodium chloride (NaCl) as a pore-forming agent was sieved to a size of 100 to 500 $\mu$m, and the hydroxyapatite powder and the sieved sodium chloride were dry-mixed in a weight ratio of 1:1 to 1.5.

The mixed powder was placed in a mold and pressurized to prepare a molded body. The prepared molded body was placed in distilled water and heated for 2 to 4 hours to remove sodium chloride in the molded body.

The hydrothermally treated molded body was dried at 100°C for 12 to 24 hours, and the dried molded body was pulverized to 0.25 to 2 mm to prepare particles.

Impurities were removed by placing the particles in a sieve, immersing them in distilled water, and flowing distilled water for 4 to 8 hours.

The washed particles were dried at 110°C for 12 to 24 hours to prepare a bone graft material.

Comparative Example 2

A bone graft material was prepared in the same manner as in Example 1, except that the silver precursor solution was prepared by dissolving silver nitrate so that the silver ion content of the final product, the bone graft material, was 1.9% by weight.

Experimental Example 1: Evaluation of Antibacterial Property

5% and 10% of Streptococcus oralis were attached to each of the bone graft materials prepared in Examples 1 to 5 and Comparative Example 1 for 3 minutes, and then removed.

The bone graft material to which bacteria were attached was immersed in a culture medium and cultured at 37°C for 7 hours.

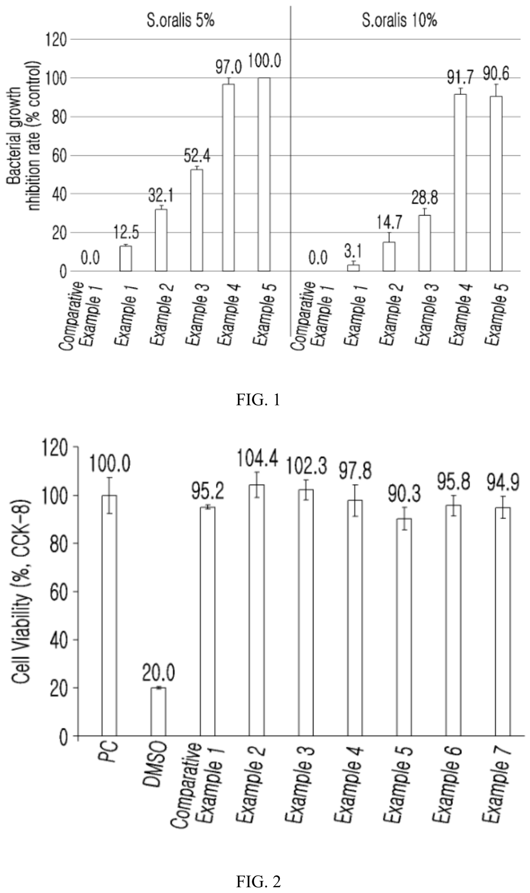

Thereafter, the optical density at 595 nm ($O.D_{595}$) was measured, and the bacterial growth inhibition rate was calculated based on the bone graft material of Comparative Example 1. The results are shown in FIG. 1.

Referring to FIG. 1, it was confirmed that the bone graft materials of Examples 1 to 5 to which silver was added exhibited a bacterial growth inhibition effect compared to the bone graft material of Comparative Example 1 to which silver was not added.

In particular, the bone graft materials of Examples 4 and 5, in which the silver ion contents were 0.6% by weight and 0.7% by weight relative to calcium ions, respectively, exhibited an excellent bacterial growth inhibition rate of 90% or more compared to the bone graft material of Comparative Example 1 to which silver was not added.

Experimental Example 2: Evaluation of Cytotoxicity

The eluates of the bone graft materials prepared in Examples 2 to 7 and Comparative Example 1 were dispensed to fibroblasts L929 and cultured for 24 hours, and then cell viability was confirmed using the CCK-8 assay.

The results are shown in FIG. 2.

Referring to FIG. 2, the bone graft materials of Examples 2 to 7 and Comparative Example 1 all exhibited high cell viability of 90% or more, confirming that there is no cytotoxicity.

Experimental Example 3: XRD Analysis

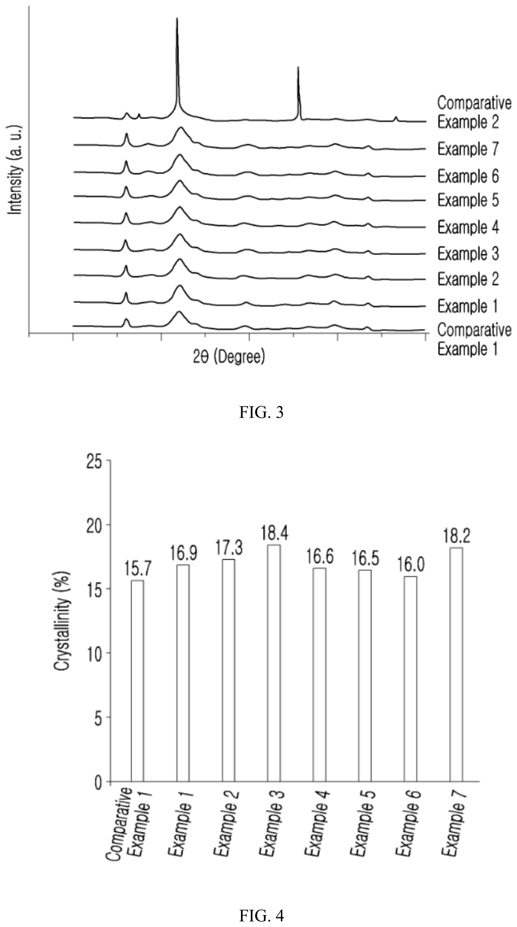

In order to confirm the crystal structure of the bone graft materials prepared in Examples 1 to 7 and Comparative Examples 1 and 2, X-ray diffraction (XRD) analysis according to ISO 13779-3 standard was performed, and the results are shown in FIG. 3.

In addition, the values obtained by XRD analysis were substituted into the following Equation 1 to calculate crystallinity, and the results are shown in FIG. 4.

<Equation 1>

$$Crystallinity = \frac{\text{Sum of 10 peak intensities of sample}}{\text{Sum of 10 peak intensities of HA standard material}} \times 100$$

Referring to FIG. 3, it was confirmed that there was no significant difference in crystal structure between the bone graft materials of Examples 1 to 7 and the bone graft material of Comparative Example 1 to which silver was not added.

On the other hand, it was confirmed that in the bone graft material of Comparative Example 2 having a silver ion content of 1.9% by weight, a secondary phase was generated in addition to the low-crystalline calcium phosphate.

Referring to FIG. 4, it was confirmed that the bone graft materials of Examples 1 to 7 and Comparative Example 1 all had low crystallinity of 25% or less.

Experimental Example 4: SEM/EDS Analysis

In order to confirm whether the silver component exists in a particle phase or is substituted in the apatite crystal, the bone graft materials prepared in Examples 1 to 6 were photographed with a scanning electron microscope (SEM), and energy-dispersive X-ray spectroscopy (EDS) was performed.

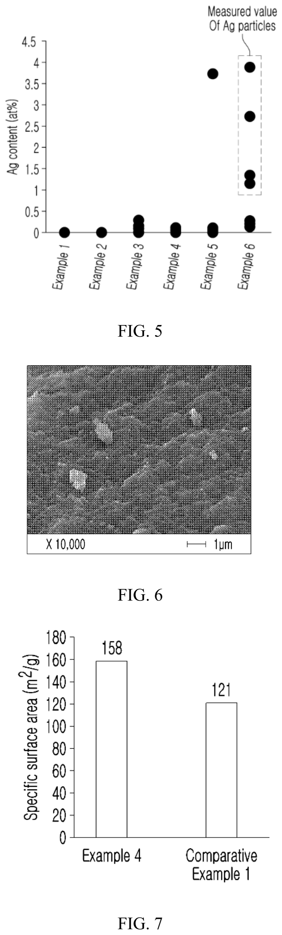

The EDS analysis results are shown in FIG. 5 and Table 1 below. FIG. 6 is an SEM image of the bone graft material prepared in Example 6.

[Table 1]

| Ag Content (at%) | Example 1 | Example 2 | Example 3 | Example 4 | Example 5 | Example 6 |
|---|---|---|---|---|---|---|
| 1 | 0 | 0 | 0.06 | 0.11 | 0.07 | 3.9 |
| 2 | 0 | 0 | 0.28 | 0.08 | 0.08 | 2.74 |
| 3 | 0 | 0 | 0.09 | 0.1 | 0.07 | 1.35 |
| 4 | 0 | 0 | 0.15 | 0.07 | 0.09 | 1.16 |
| 5 | 0 | 0 | 0.1 | 0.07 | 0.12 | 0.14 |
| 6 | 0 | 0 | 0 | 0.1 | 0.15 | 0.29 |
| 7 | 0 | 0 | 0 | 0.07 | 3.73 | 0.23 |

Referring to FIG. 5, silver component particles were not present in the bone graft materials of Examples 1 to 4, and a small amount of silver particles were present in the bone graft material of Example 5.

Referring to Table 1, silver component particles of significant concentration did not appear in Examples 1 to 4, but in Example 5, some silver particles were crystallized outside the bone graft material to show high Ag content, and it was confirmed that this tendency was more pronounced in Example 6.

In particular, referring to FIGS. 5 and 6, crystallized silver particles were present in the bone graft material of Example 6 having a high silver ion content of 1.0% by weight. Through this, it was confirmed that the silver component added in Examples 1 to 6 exists by being substituted in the apatite crystal, but if the amount of silver added increases, saturated silver particles cannot be substituted into the lattice and are crystallized.

Experimental Example 5: ICP Analysis

In order to confirm the silver ion content contained in the bone graft material, Inductively Coupled Plasma (ICP) analysis was performed on the bone graft materials prepared in Examples 1 to 7 and Comparative Examples 1 and 2.

The results are shown in Table 2 below. The silver ion content (% by weight) below is calculated and expressed as a weight ratio of silver ions to total elements constituting the bone graft material.

[Table 2]

| Component | E1 | E2 | E3 | E4 | E5 | E6 | E7 | CE1 | CE2 |
|---|---|---|---|---|---|---|---|---|---|
| Silver ion content (wt%) | 0.2 | 0.3 | 0.4 | 0.6 | 0.7 | 1.0 | 1.5 | 0 | 1.9 |
| * E: Example / CE: Comparative Example | | | | | | | | | |

Referring to Table 2, it was confirmed that the bone graft materials of Examples 1 to 7 and Comparative Example 2 all exhibited the target silver ion content.

The silver ion content contained in the bone graft materials of Examples 1 to 7 increased in proportion to the amount of silver added.

Experimental Example 6: Specific Surface Area Analysis

The specific surface area of the bone graft materials prepared in Example 4 and Comparative Example 1 was analyzed according to the BET method using nitrogen gas, and the results are shown in FIG. 7.

Referring to FIG. 7, it was confirmed that the bone graft materials of Example 4 and Comparative Example 1 both exhibited a high BET specific surface area of 100 $m^2$/g or more.

The above description of the present disclosure is for specific illustration, and those skilled in the art to which an aspect of the present disclosure pertains will understand that it can be easily modified into other specific forms without changing the technical spirit or essential features described in the present disclosure.

Therefore, the examples described above should be understood as illustrative in all respects and not restrictive.

For example, each component described as a single type may be implemented in a distributed manner, and similarly, components described as being distributed may be implemented in a combined form.

The scope of the present disclosure is indicated by the claims to be described later, and all changes or modified forms derived from the meaning and scope of the claims and their equivalent concepts should be construed as being included in the scope of the present disclosure.

**Claims**

1. A method for preparing a low-crystalline calcium phosphate-based antibacterial bone graft material, the method comprising:

    (a) synthesizing calcium phosphate by adding a calcium precursor and a phosphate precursor all at once;
    (b) introducing the calcium phosphate into an antibacterial material precursor solution to perform a reaction;
    (c) freeze-drying a product of step (b);
    (d) mixing a product of step (c) with a pore-forming agent and press-molding the mixture to prepare a molded body; and
    (e) removing the pore-forming agent of the molded body in a solvent at a temperature equal to or higher than the boiling point of the solvent, followed by drying.

2. The method of claim 1, wherein the calcium precursor comprises at least one selected from the group consisting of calcium nitrate, calcium chloride, calcium fluoride, calcium iodide, calcium acetate, ammonium carbonate, and ammonium bicarbonate.

3. The method of claim 1, wherein the phosphate precursor comprises at least one selected from the group consisting of phosphorus oxide, ammonium phosphate monobasic, ammonium phosphate dibasic, ammonium phosphate tribasic, sodium phosphate monobasic, sodium phosphate dibasic, sodium phosphate tribasic, and potassium phosphate.

4. The method of claim 1, wherein in step (a), a molar ratio of the calcium precursor and the phosphate precursor is 1:1.5 to 3.

5. The method of claim 1, wherein step (a) is a wet precipitation method in which each precursor is dissolved in distilled water and mixed.

6. The method of claim 1, wherein the antibacterial material comprises at least one selected from the group consisting of silver (Ag), magnesium (Mg), gallium (Ga), zinc (Zn), and copper (Cu).

7. The method of claim 1, wherein the reaction of step (b) is performed at a temperature of 15 to 25°C for 10 to 20 hours.

8. The method of claim 1, wherein in step (d), a weight ratio of the product of step (c) and the pore-forming is 1:1 to 1.5.

9. The method of claim 1, wherein an average particle diameter of the pore-forming is 100 to 500 $\mu$m.

10. The method of claim 1, further comprising, after step (e): (f) heat-treating the molded body at a temperature of 250°C or lower.

11. The method of claim 1, wherein a content of an antibacterial material in the low-crystalline calcium phosphate-based antibacterial bone graft material is 0.2 to 1.8% by weight.

12. The method of claim 1, wherein a content of an antibacterial material in the low-crystalline calcium phosphate-based antibacterial bone graft material is 0.5 to 0.9% by weight.

13. A low-crystalline calcium phosphate-based antibacterial bone graft material comprising an antibacterial material, wherein the bone graft material includes at least one of grooves or pores having an average diameter of 100 to 500 $\mu$m, wherein the bone graft material has a BET specific surface area of 100 $m^2$/g or more, wherein the bone graft material includes both crystalline apatite and amorphous calcium phosphate, and wherein the bone graft material has a crystallinity of 25% or less.

14. The low-crystalline calcium phosphate-based antibacterial bone graft material of claim 13, wherein the antibacterial material comprises at least one selected from the group consisting of silver (Ag), magnesium (Mg), gallium (Ga), zinc (Zn), and copper (Cu).

15. The low-crystalline calcium phosphate-based antibacterial bone graft material of claim 13, wherein a content of the antibacterial material is 0.2 to 1.8% by weight relative to total elements constituting the bone graft material.

16. The low-crystalline calcium phosphate-based antibacterial bone graft material of claim 13, wherein a content of the antibacterial material is 0.5 to 0.9% by weight relative to total elements constituting the bone graft material.

FIG. 1

FIG. 2

FIG. 3

FIG. 4

FIG. 5

FIG. 6

FIG. 7

## INTERNATIONAL SEARCH REPORT

| International application No. |
|---|
| **PCT/KR2024/007005** |

| | |
|---|---|
| **A.** | **CLASSIFICATION OF SUBJECT MATTER** |
| | **A61L 27/12**(2006.01)i; **A61L 27/04**(2006.01)i |

According to International Patent Classification (IPC) or to both national classification and IPC

| | |
|---|---|
| **B.** | **FIELDS SEARCHED** |

Minimum documentation searched (classification system followed by classification symbols)

A61L 27/12(2006.01); A01N 59/06(2006.01); A01N 61/00(2006.01); A61L 27/46(2006.01); A61L 27/56(2006.01); C01B 25/32(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 항균 골 이식재(antibiotic bone graft material), 저결정성 칼슘인산염(low crystalline calcium phosphate salt), 금속 항균 물질(metal antibiotic material), 동결건조(lyophilization), 형상조절제(shape controller), 마이크로포어(micropore), 비표면적(specific surface area)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| Y | KR 10-0489549 B1 (ETEX CORPORATION) 31 October 2005 (2005-10-31)<br>See paragraphs [0011]-[0060], [0073] and [0092]; and claims 1 and 4. | 1-16 |
| Y | KR 10-0941511 B1 (KOREA ATOMIC ENERGY RESEARCH INSTITUTE) 10 February 2010 (2010-02-10)<br>See paragraphs [0006]-[0024] and [0040]; claim 1; and figure 2. | 1-16 |
| Y | KR 10-2005-0050024 A (T&LIFE SYSTEM LTD.) 27 May 2005 (2005-05-27)<br>See abstract; and paragraph [0007]. | 6,14 |
| A | US 2015-0024023 A1 (THE UNIVERSITY COURT OF THE UNIVERSITY OF ABERDEEN et al.) 22 January 2015 (2015-01-22)<br>See entire document. | 1-16 |

| | |
|---|---|
| ✓ Further documents are listed in the continuation of Box C. | ✓ See patent family annex. |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 September 2024** | **06 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2024/007005** |

### C. DOCUMENTS CONSIDERED TO BE RELEVANT

| Category* | Citation of document, with indication, where appropriate, of the relevant passages | Relevant to claim No. |
|---|---|---|
| A | MOHAMMAD, Nur Farahiyah et al. Synthesis & characterization of mesoporous hydroxyapatite by co-precipitation method. Asian International Conference on Materials, Minerals and Polymer 2012. 23-24 March 2012, pp. 626-633.<br>See entire document. | 1-16 |

Form PCT/ISA/210 (second sheet) (July 2022)

**INTERNATIONAL SEARCH REPORT**
Information on patent family members

International application No.

**PCT/KR2024/007005**

| Patent document cited in search report | | | Publication date (day/month/year) | Patent family member(s) | | | Publication date (day/month/year) |
|---|---|---|---|---|---|---|---|
| KR | 10-0489549 | B1 | 31 October 2005 | CA | 2236572 | A1 | 15 May 1997 |
| | | | | CA | 2236572 | C | 08 April 2008 |
| | | | | EP | 859737 | A1 | 26 August 1998 |
| | | | | EP | 859737 | B1 | 31 August 2011 |
| | | | | JP | 2000-500110 | A | 11 January 2000 |
| | | | | JP | 4117803 | B2 | 16 July 2008 |
| | | | | KR | 10-1999-0067360 | A | 16 August 1999 |
| | | | | US | 5783217 | A | 21 July 1998 |
| | | | | WO | 97-17285 | A1 | 15 May 1997 |
| KR | 10-0941511 | B1 | 10 February 2010 | KR | 10-2009-0051857 | A | 25 May 2009 |
| KR | 10-2005-0050024 | A | 27 May 2005 | KR | 10-0647220 | B1 | 23 November 2006 |
| US | 2015-0024023 | A1 | 22 January 2015 | EP | 2794471 | A1 | 29 October 2014 |
| | | | | EP | 2794471 | B1 | 07 February 2018 |
| | | | | ES | 2665848 | T3 | 27 April 2018 |
| | | | | JP | 2015-502327 | A | 22 January 2015 |
| | | | | JP | 6396803 | B2 | 26 September 2018 |
| | | | | NO | 2794471 | T3 | 07 July 2018 |
| | | | | US | 9492585 | B2 | 15 November 2016 |
| | | | | WO | 2013-093439 | A1 | 27 June 2013 |

Form PCT/ISA/210 (patent family annex) (July 2022)